(19) Europäisches Patentamt
European Patent Office
Office européen des brevets

(11) Publication number: **0 123 303 A1**

(12) **EUROPEAN PATENT APPLICATION**

(21) Application number: 84104510.7

(22) Date of filing: 19.04.84

(51) Int. Cl.³: C 07 D 405/12
C 07 D 307/79, A 01 N 47/36

(30) Priority: 21.04.83 IT 2070783

(43) Date of publication of application: 31.10.84 Bulletin 84/44

(84) Designated Contracting States: BE CH DE FR GB LI NL

(71) Applicant: Montedison S.p.A.
31, Foro Buonaparte
I-20121 Milan(IT)

(72) Inventor: Gozzo, Franco, Dr.
52/A, via Triulziana
S. Donato Milanese (Milano)(IT)

(72) Inventor: Paolucci, Paride, Dr.
8, via Trieste
S. Donato Milanese (Milano)(IT)

(72) Inventor: Preziuso, Ciro
29, via Bessarione
Milano(IT)

(74) Representative: Schmied-Kowarzik; Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Dihydro-benzofuran derivatives having herbicide activity.**

(57) There are disclosed compounds of formula:

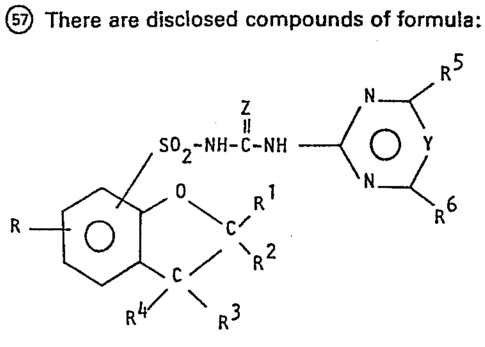

(I)

wherein:

R = H, a halogen atom, $CH_3$, $NO_2$, a COO-alkyl, a $SO_2$-alkyl, an aminocarbonyl, an O-alkyl, an O-alkenyl, an O-alkynyl, an O-aryl, a $NR^7-CO-R^8$ group ($R^7$ and $R^8$ = H, alkyl group);

$R^1$, $R^2$, $R^3$ and $R^4$ = H, $CH_3$ or $R^1$ and $R^2$ together form with the carbon atom, they are linked to, carbonyl group and/or $R^3$ and $R^4$ together, form with the carbon atom, they are linked to, a carbonyl group;

Z = O, S;
Y = CH, N;
$R^5$ and $R^6$ = $CH_3$, $OCH_3$.

The compounds of formula I are endowed with a high herbicide activity.

EP 0 123 303 A1

The present invention relates to compounds having herbicide activity and more precisely it relates to dihydro-benzofuran derivatives having herbicide activity.

The compounds, which form an object of the present invention have general formula:

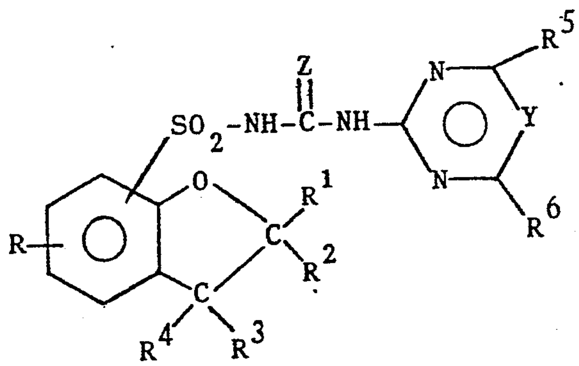

(I)

wherein:

R represents a hydrogen atom, a halogen atom, a methyl, a nitro, a COO-alkyl, a $SO_2$-alkyl, an aminocarbonyl, a mono- or dialkylcarbonyl group, an O-alkyl group optionally sub= stituted by from 1 to 3 halogen atoms, an O-alkenyl group optionally substituted by from 1 to 3 halogen atoms, an O-alkynyl, an O-aryl or a $NR^7$-CO-$R^8$ group, wherein $R^7$ an $R^8$ are independently a hydrogen atom, an alkyl or a phenyl group or together they form a trimethylenic or a tetrame= thylenic bridge;

$R^1$, $R^2$, $R^3$ and $R^4$, equal to or different from each other, re= present a hydrogen atom or a methyl group; or

$R^1$ and $R^2$ together with the carbon atom, they are linked to, form a carbonyl group (C=O) and/or $R^3$ and $R^4$ together with the carbon atom, they are linked to, form a carbonyl group (C=O);

$R^5$ and $R^6$, equal to or different from each other, represent a methyl or a methoxy group;

Z represents an oxygen or a bivalent sulphur atom;

Y represents a nitrogen atom or a CH group;

with alkyl group an alkyl group is meant having from 1 to 4 carbon atoms; with alkenyl group an alkenyl group having from 2 to 5 carbon atoms; with alkynyl group an alkynyl group ha= ving from 2 to 5 carbon atoms, with aryl group a phenyl group optionally substituted by from 1 to 3 halogen atoms or alkyl groups, a 2-pyridil group; with halogen atom a fluorine, chlo= rine, bromine or iodine atom.

The compounds of formula I are endowed with herbicide activi= ty and are fit for being used in the agrarian field for pro= tecting useful cultivations against weeds.

Examples of compounds of formula I are the following:

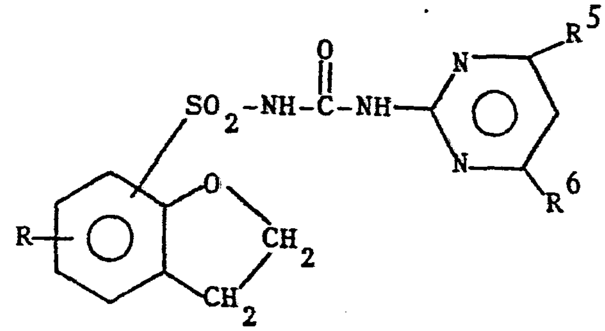

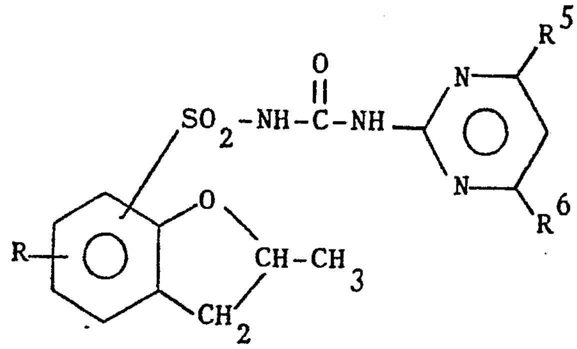

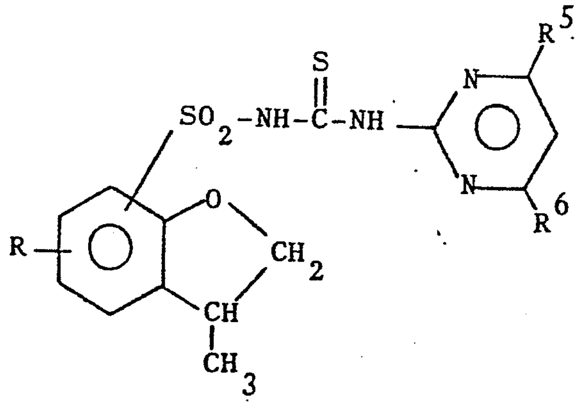

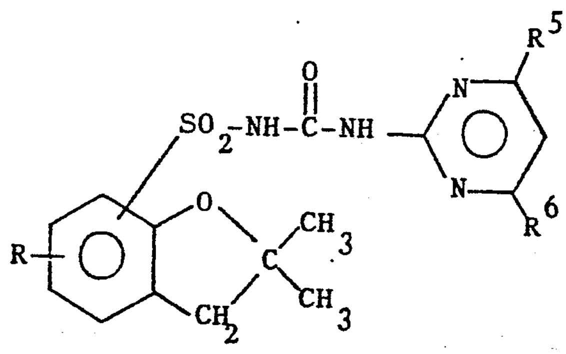

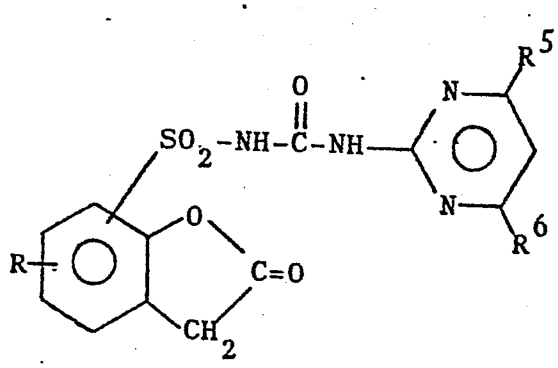

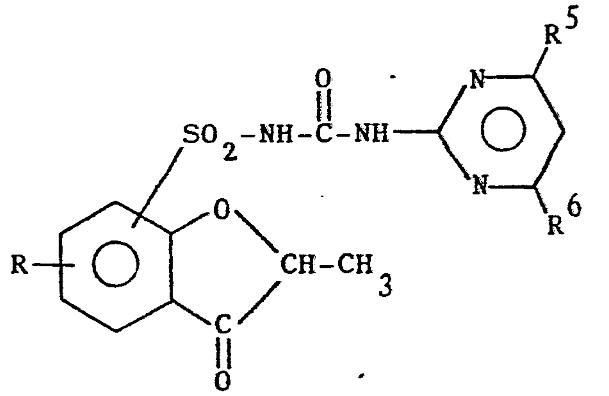

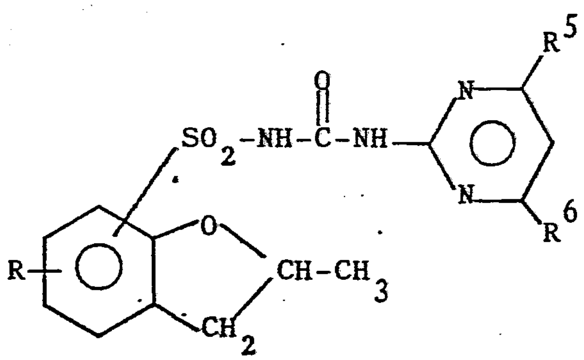

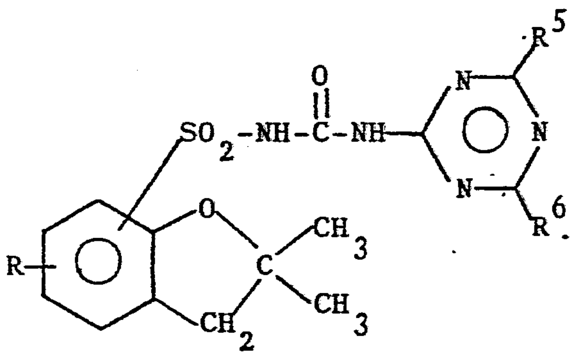

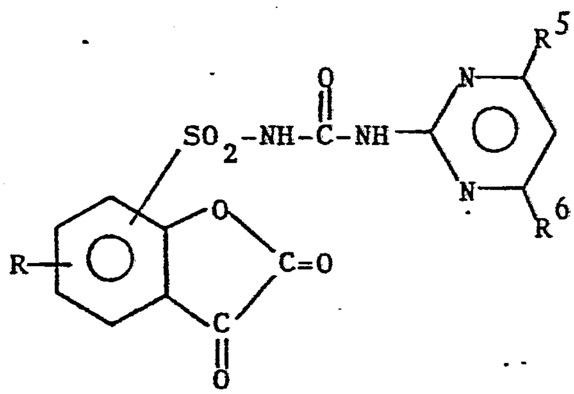

wherein R, $R^5$ and $R^6$ have the meanings recorded for formula I

The preparation of the compounds of formula I is carried out according to the reactions reported in following scheme 1 and remarked hereinafter.

Scheme 1

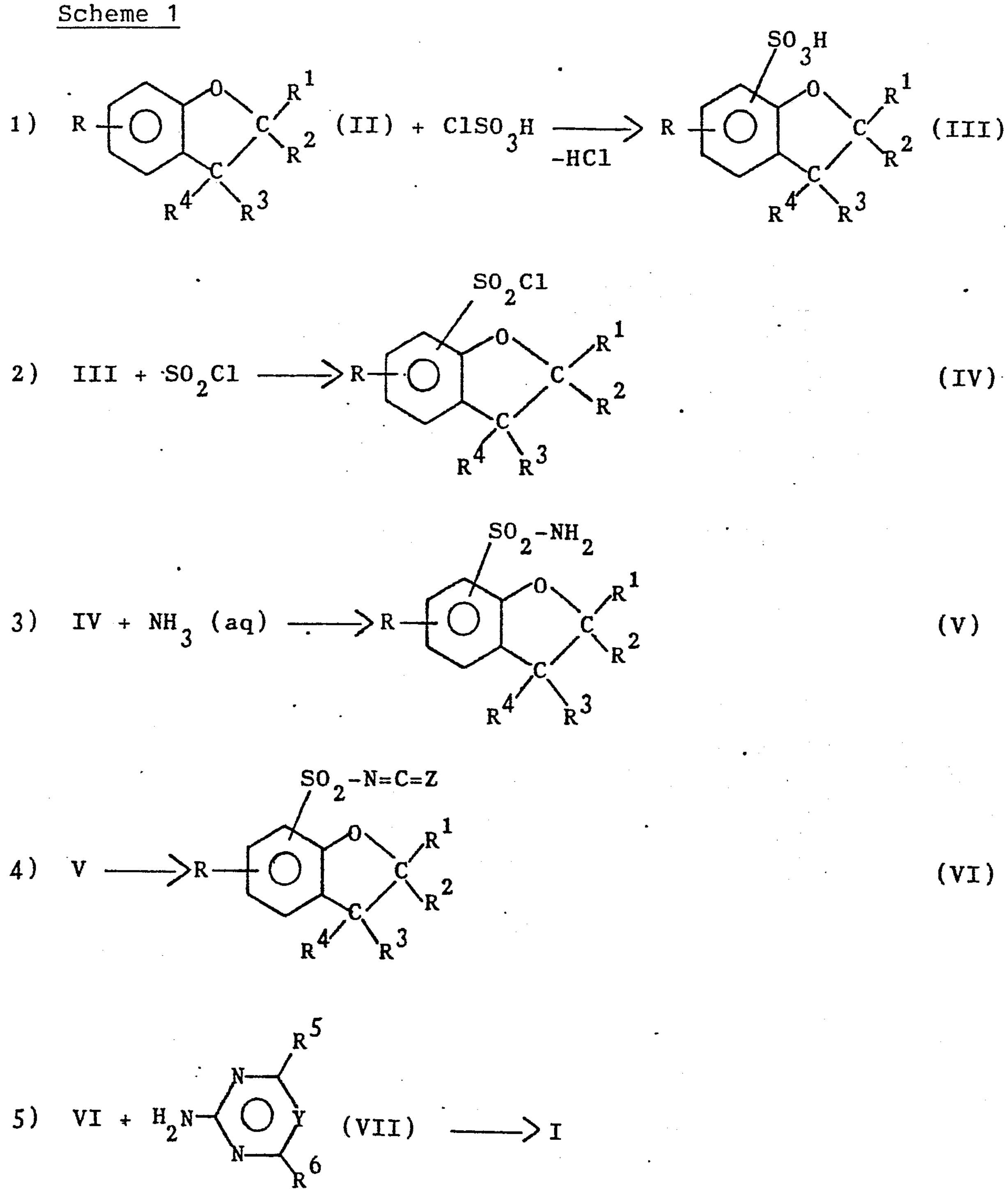

($R^1$, $R^2$, $R^3$, $R^4$, $R^5$, $R^6$, Y and Z have the same meaning recor= ded for formula I).

Reaction 1 of scheme 1 consists in the sulfonation of the dihy= dro-benzofuran of formula II.

Reaction 1 can be carried out according to different techni= ques, which are known in literature for the sulfonation of aro= matic nuclei. A method that has given good results, consists in reacting the compound of formula II with chlorosulfonic acid in an inert solvent such as chloroform, at a temperature ranging between -10 and 40°C.

Other literature methods that can be applied, are the ones de= scribed by R.V. Hoffmann in "Organic Synthesis" (O.L.Chapman publisher) volume 60, pages 121-126 (1981) and by H.A. Young in Journal Am. Chem. Soc. 59, 811 (1937).

The benzo-furan-sulfonic acids of formula III are then converted into the corresponding sulfonic chlorides (Reaction 2) and from these into the sulfonamides of formula V (Reaction 3) which are new compounds.

Both reaction 2 and reaction 3 are carried out according to modalities, which are well known in literature. The conver= sion of the sulfonamides of formula V into the corresponding isocyanates (or isothiocyanates) of formula VI (Reaction 4) can be carried out according to different known techniques. Among these techniques the following ones can be cited:

- the reaction of sulfonamides with an alkyl-isocyanate followed by phosgenation of the resulting urea [H.Ulrich et al, J. Org. Chem. 31, 2658 (1966)];
- the reaction of sulfonamides with chlorosulfonyl-isocyanate [R. Appel et al, Chem. Ber. 107, 706 (1974)];
- the reaction of sulfonamides with oxalyl chloride and subsequent pyrolysis of the resulting sulfonyl-oxaloyl-chlorides in dichlorobenzene [J.E. Franz et al, J. Org. Chem. 29, 2592 (1964)].

The first two processes are burdensome owing to the cost of the employed reactants and owing to the long operations required to isolate the product.

Should the sulfonamide not be soluble in oxalyl chloride, the third process could not be applied under the conditions, as described be the author.

Therefore it was necessary, in the present case, to modify the operational conditions described by J. E. Franz, by reacting the sulfonamide with an excess of oxalyl chloride in ortho-dichlorobenzene at temperatures comprised between 90 and 180°C.

The excess of oxalyl chloride is removed by distillation as reaction goes on, till the distillation temperature of the solvent is reached. Finally reaction 5 of scheme 1 is carried out by reacting isocyanate VI with aromatic amine VII suspended

in an inert solvent such as acetonitrile or methylene chlo=ride at room temperature and in the presence of catalytic amounts of a tertiary amine.

The starting products of the reactions of Scheme 1, and na=mely the dihydro-benzofuran derivatives of formula II, are known compounds or they can be prepared according to known techniques. The preparation of some compounds of formula II can require a specific synthesis, which will be illustra=ted shortly hereinafter, for the following structures:

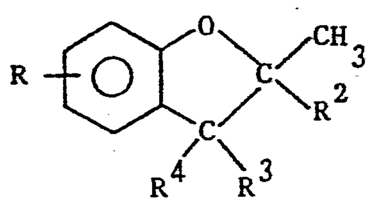

(II-A, $R^2$, $R^3$ ed $R^4$ = H, $CH_3$)

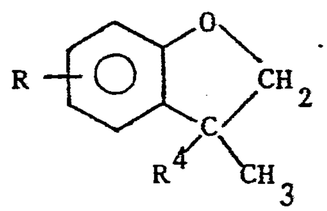

(II-B, $R^4$ = H, $CH_3$)

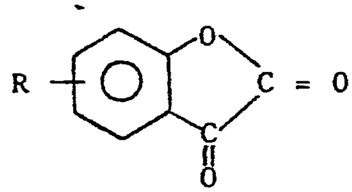

(II-C)

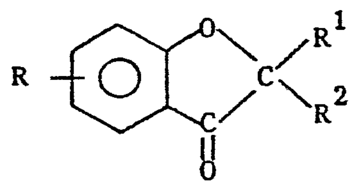

(II-D, $R^1$ ed $R^2$ = H, $CH_3$)

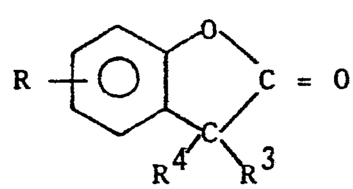

(II-E, $R^3$ ed $R^4$ = H, $CH_3$)

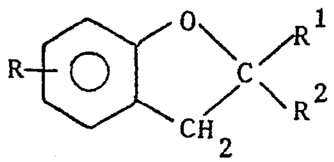 (II-F, $R^1$ ed $R^2$ = H, $CH_3$)

The preparation of the compounds of formula II-A is carried out by means of the following reactions:

6) 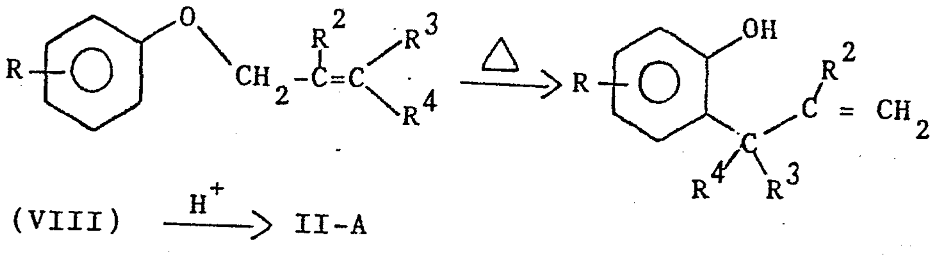

(VIII)

7) (VIII) $\xrightarrow{H^+}$ II-A

Reaction 6 is a Claisen transposition that is carried out, for instance, according to what is described in "Organic Reactions", vol. II, chapter 1° (1944) and vol. 22°, chapter 1° (1975).

The cyclization of intermediate VIII (Reaction 7) was de= scribed in "Organic Reactions" vol. II, chapter 1° (1944). In such a reference one states to use hydrobromic acid or formic acid as catalyst.

According to our present experience we state as more conve= nient, at least in some cases (in particular when $R^2 = CH_3$), to carry out reactions 6 and 7 in only one stage, by heating the starting products between 150 and 220°C in the presence of catalytic amounts of magnesium chloride.

The preparation of the compounds of formula II-B can be

0123303

carried out starting from the compounds of formula

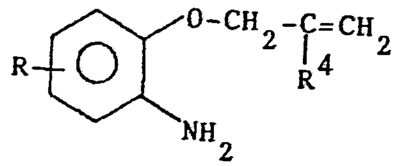

(IX)

through transformation of amino group into diazo and radi= calic cyclization of diazo-derivative in the presence of tributyl-tin hydride $(n.C_4H_9)_3$ Sn H according to the me= thod described by A.L.J. Beckwith et al, J. Chem. Soc. - Chem. Comm. 136 (1981).

The compounds of formula II-C are prepared from compounds of formula

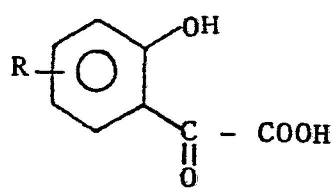

by dehydration and cyclization according to methods known in literature, for instance Fries, Pfaffendorf; Berichte 45, 156 (1912).

The preparation of compounds of formula II-D can be carried out by internal condensation of the compounds of formula (X) according to reaction 8:

8) 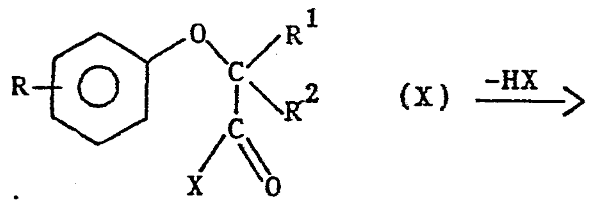 (X) -HX II-D

wherein X = OH or a halogen atom (Cl, Br).

Reaction 8 can be carried out under the conditions described by Fries, Hasselbach e Schroeder, Angewandte <u>405</u>, 370 (1914).

Alternatively the compounds of formula II-D can be prepared by condensing the suitable phenol with the chloride of a 2-chloro-alkanoyc acid (XI) under Friedel and Crafts conditions and by cyclizing the resulting product (XII) in the presence of a base according to the following reactions:

9) 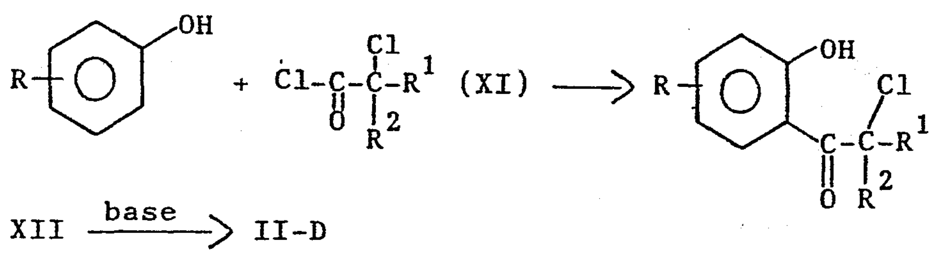
(XI) (XII)

10) XII
base
-HCl
II-D

The compounds of formula II-E can be prepared, according to procedures analogous to the ones described for the preparation of compounds II-C, by intemolecular condensation of the corresponding 2-(2-hydroxyfuryl)-alkanoyc acids of formula

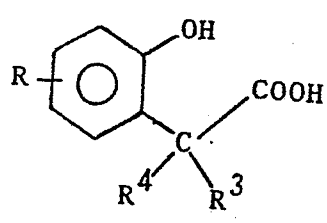
(XIII)

Finally the preparation of the compounds of formula II-F can be carried out by catalytic hydrogenation of the carbonyl group of the compounds of formula II-D to methylene.

As hereinbefore reported, the compounds of formula I are endowed with a high herbicide activity and other positive characteristics, which allow the use of such compounds in the agrarian field for protecting useful cultivations against weeds.

These characteristics can be summarized in a high and ver= satile herbicide action, in a wide spectrum of action and in the selectivity towards agrarian cultivations.

In fact, the compounds of formula I are active towards a lot of species of weeds, in particular against the dicoty= ledons and exhibit a herbicide activity both during the pre-emergence treatments and, mostly, during the post-emergen= ce treatments.

In the meanwhile the compounds of formula I prove to be se= lective towards important agrarian cultivations such as the cereals.

For the practical uses in agriculture the compounds, object of the invention, may be used as such or, according to the normal practice, in the form of a suitable composition.

In the compositions, besides the compound of formula I as active substance, inert carriers may be present, which may be both solid and liquid and optionally other additives of agrarian use.

According to the normal formulative practice the compositions

may be in the form of liquid concentrates, emulsifiable concentrates, suspensions, formulates in powder or in wet=table powder and granular formulates.

If desired, to deal with specific situations it is possi=ble to add to the compositions other active substances use=ful in agriculture such as fertilizers, fungicides or other weed-killers.

The amount of compound of formula I to be used for protec=ting useful cultivations against the weeds depends on dif=ferent factors. Among these we can cite the kind and the degree of infestation, the kind of treatment (pre-emergen=ce or post-emergence), the respective effectiveness of the specific product of formula I, that is used, also in func=tion of the above mentioned factors, the kind of cultiva=tion to be weedded and climatic and environmental factors.

In general, satisfactory results are obtained by using an amount of compound of formula I ranging between 0.1 and 3 Kg/ha.

The invention will be now better illustrated by the follo=wing examples.

EXAMPLE 1

Preparation of 2,3-dihydro-2,2-dimethyl-5-nitro-7-benzofuransulfonic acid.

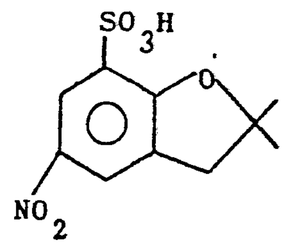

A solution consisting of 6,4 g of chlorosulfonic acid in 3 ml of anydrous chloroform was added dropwise to a solu=tion containing 9.7 g of 2,3 dihydro-2,2-dimethyl-5-nitro=benzofuran in 10 ml of anydrous chloroform maintained un=der good stirring at the temperature of 0 - 3 °C. On com=pletion of the addition the temperature of the reacting mix=ture was allowed to rise gradually to 20°C, then one heated for two hours at 40°C. By cooling a solid precipitated, which was separated by filtration and washed with chloroform.
The obtained product, after having been dried under vacuum, consisted of a yellow solid (4.9 g) having a M.P.: 180-182°C.
IR was consistent with the assigned structure.

EXAMPLE 2

Preparation of 2,3-dihydro-2,2-dimethyl-5-nitro-7-benzofuran=sulfonylchloride

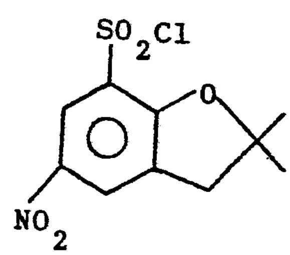

9 g of 2,3-dihydro-2,2-dimethyl-5-nitro-7-benzofuransulfonic

acid and 0.02 g of N,N-dimethylformamide were added to a solution containing 39.3 g of thionyl chloride in 25 ml of toluene.

The mixture was maintained under reflux for three hours; at the end a solution was obtained from which by cooling a so= lid crystallized that was separated by filtration, washed with toluene and dried in the air till a constant weight was reached.

The thus obtained product consisted of 6.6 g of a white crystalline solid having a M.P.: 154-155°C.

IR was consistent with the assigned structure.

EXAMPLE 3

Preparation of 2,3-dihydro-2,2-dimethyl-5-nitro-7-benzofuran= sulfonamide

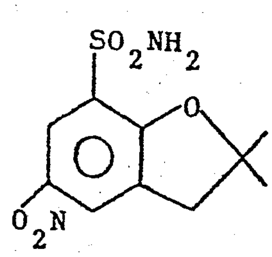

A mixture consisting of 5.6 g of 2,3-dihydro-2,2-dimethyl-5-nitro-7-benzofuransulfonylchloride, 35 ml of a solution at 32% of ammonium hydroxide and 100 ml of toluene was heated under a strong stirring for four hours. When this period of time had elapsed, the reaction mixture was cooled down to 5°C and the solid in suspension was separated by filtra= tion, washed with water and dried in the air till a constant

weight was reached .

The thus obtained product consisted of 4.3 g of a crystal= line solid having a M.P.: 195-197°C.

IR and NMR spectra were consistent with the assigned struc= ture.

EXAMPLE 4

Preparation of 2,3-dihydro-2,2-dimethyl-5-chloro-7-benzo= furansulfonic acid.

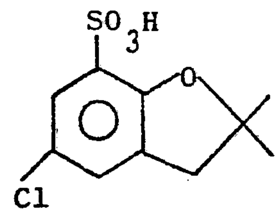

6.4 g of chlorosulfonic acid were added dropwise to a solu= tion containing 9.1 g of 2,3-dihydro-2,2-dimethyl-5-chloro-benzofuran in 20 ml of anydrous chloroform, cooled at 0-3°C and maintained under good stirring.

On completion of the addition the temperature of the reac= tion mixture was allowed to rise to 20°C always under good stirring and these conditions were still maintained for two hours; the precipitate was then filtered, washed on the fil= ter with a small amount of chloroform and rapidly put into a drier under vacuum till a constant weight was reached.

The obtained product, 10.5 g was in the form of a white cry= stalline solid having a M.P.: 169-171°C.

IR was consistent with the assigned structure.

EXAMPLE 5

Preparation of 2,3-dihydro-2,2-dimethyl-5-chloro-7-benzo= furansulfonylchloride

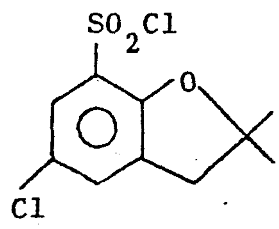

A mixture containing 5.2 g of 2,3-dihydro-2,2-dimethyl-5-chloro-7-benzofuransulfonic acid and 15.5 g of phosphorus oxychloride was kept at 80-85°C under good stirring for four hours. The obtained solution, after having been cooled down, was added with 100 ml of dichloroethane and poured into 500 ml of a mixture of water and ice.

The organic phase, after having been separated, washed with water, dehydrated and evaporated in a rotary evaporator under reduced pressure, provided a residue consisting of 4.3 g of a white solid having a M.P.:104-106°C.

IR was consistent with the assigned structure.

EXAMPLE 6

Preparation of 2,3-dihydro-2,2-dimethyl-5-chloro-7-benzofuran= sulfonamide.

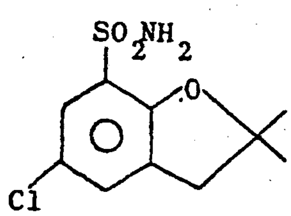

A solution containing 2.8 g of 2,3-dihydro-2,2-dimethyl-5-chloro-7-benzofuransulfonylchloride in 30 ml of toluene was added slowly to 30 ml of a solution at 32% of ammonium hydroxide under good stirring.

The mixture was heated at 100°C for seven hours, then cooled down and the solid precipitate was separated by filtration, washed with water and dried in the air, till a constant weight was reached. 2.3 g of product were obtained, having a M.P.: 188-190°C.

IR and NMR spectra were consistent with the assigned structure.

EXAMPLE 7

Some 2,3-dihydro-benzofuransulfonamides of formula are recorded on following Table 1

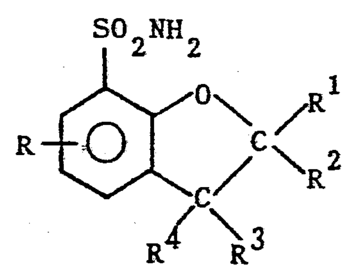

(V)

| (a) Compound | R | $R^1$ (or $R^2$) | $R^2$ (or $R^1$) | $R^3$ (or $R^4$) | $R^4$ (or $R^3$) | Melting point (°C) (b) |
|---|---|---|---|---|---|---|
| A (c) | 5-$NO_2$ | $CH_3$ | $CH_3$ | H | H | 195-7 |
| B (d) | 5-Cl | $CH_3$ | $CH_3$ | H | H | 188-90 |
| C | 5-Cl | $CH_3$ | H | H | H | 169-71 |
| D | 5-$NO_2$ | $CH_3$ | H | H | H | 196-8 |
| E | 5-$CH_3$ | $CH_3$ | $CH_3$ | H | H | 141-2 |

Notes to Table 1

(a) IR and elemental analysis of each compound are consi=
stent with the assigned structure;

(b) the melting points were not corrected;

(c) the preparation of compound A is described in detail in
example 3;

(d) the preparation of compound B is described in detail in
example 6.

EXAMPLE 8

Preparation of 2,3-dihydro-2,2-dimethyl-5-chloro-7-benzofu=
ransulfonylisocyanate

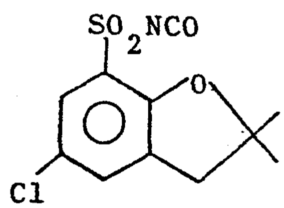

A mixture consisting of 7.3 g of 2,3-dihydro-2,2-dimethyl-5-chloro-7-benzofuransulfonamide, 18 g of oxalyl chloride and 70 ml of anhydrous o-dichlorobenzene was maintained un=
der reflux for two hours.

After this period of time the excess of oxalyl chloride was removed by distillation at normal pressure by rising the temperature till 160°C, then under reduced pressure the o-dichlorobenzene was removed by fractional distillation from the desired product. This consisted of a fraction of

0123303

6.8 g having a boiling point 153-155°C/0.06 Torr.

IR was consistent with the assigned structure.

EXAMPLE 9

Preparation of N-[(4-methoxy-6-methyl-1,3,5-triazin-2-il) aminocarbonyl]-2,3-dihydro-2,2-dimethyl-5-chloro-7-benzo=furansulfonamide

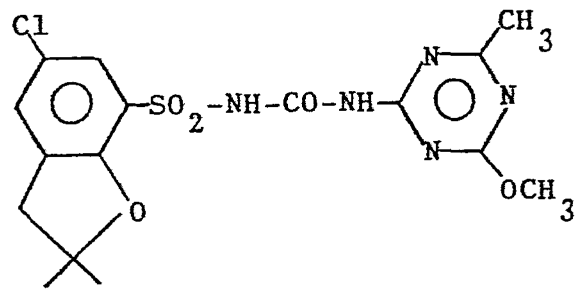

A solution consisting of 1.6 g of 2,3-dihydro-2,2-dimethyl-5-chloro-7-benzofuransulfonylisocyanate in 2 ml of anhydrous acetonitrile was added at room temperature to a well stirred suspension of 0.7 g of 2-amino-4-methoxy-6-methyl-1,3,5-triazine in 20 ml of anhydrous acetonitrile containing 0.02 g of triethylamine.

The mixture was kept under good stirring at room temperature for twenty-four hours and then was filtered in order to se=parate the residue on the bottom. The solution was evapora=ted in a rotary evaporator at 30°C under the residual pres=sure of 15 Torr.

The obtained residue, 1.2 g, was purified on a silica gel chromatographic column with toluene/ethyl acetate (15/5) as

eluent, thereby obtaining a white crystalline solid with M.P.: 170-72°C.

IR was consistent with the assigned structure.

Elemental analysis $C_{16}H_{18}ClN_5O_5S$:

| | | | |
|---|---|---|---|
| $C_{calculated}$ = | 44.90% | $C_{found}$ = | 44.95% |
| $H_{calculated}$ = | 4.24% | $H_{found}$ = | 4.27% |
| $N_{calculated}$ = | 16.37% | $N_{found}$ = | 16.03% |
| $Cl_{calculated}$ = | 8.28% | $Cl_{found}$ = | 8.39% |
| $S_{calculated}$ = | 7.49% | $S_{found}$ = | 7.22% |

<u>EXAMPLE 10</u>

Preparation of N-[(4,6-dimethyl-2-pyrimidyl)-aminocarbonyl]-
-2,3-dihydro-2,2-dimethyl-5-chloro-7-benzofuransulfonamide

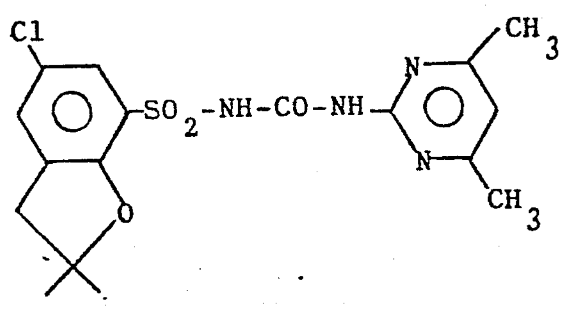

1.6 g of 2,3-dihydro-2,2-dimethyl-5-chloro-7-benzofuran=sulfonylisocyanate dissolved in 2 ml of anhydrous acetoni=trile were added at room temperature to a well stirred mix=ture consisting of 0.6 g of 4,6-dimethyl-2-amino-pyrimidine, 0.01 g of triethylamine and 15 ml of anhydrous acetonitrile. The reacting bulk was kept under stirring at room temperatu=re for 15 hours.

The formed precipitate was separated by filtration and dried in the air till a constant weight was reached.

The obtained product was in the form of a white crystalline solid, 0.9 g, with a M.P.:187-189°C.

IR was consistent with the assigned structure.

EXAMPLE 11

Preparation of N-[(4-methoxy-6-methyl-1,3,5-triazin-2-il)-aminocarbonyl]-2,3-dihydro-2,2,5-trimethyl-7-benzofuran=sulfonamide

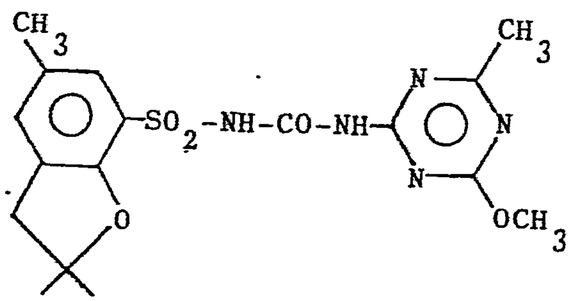

A solution consisting of 1.5 g of 2,3-dihydro-2,2,5-trime=thyl-7-benzofuransulfonylisocyanate in 3 ml of anhydrous acetonitrile was added at room temperature to a well stirred suspension of 0.7 g of 2-amino-4-methoxy-6-methyl-1,3,5-triazine in 10 ml of anhydrous acetonitrile containing 0.02 g of triethylamine.

The mixture was kept under good stirring at room temperature for thirty-six hours, then was filtered in order to separate the residue on the bottom. The solution was evaporated in a rotary evaporator at 30°C under reduced pressure (15 Torr) till 1.5 g of solid residue were obtained.

The crude product was dissolved in toluene and the obtained solution, after separation by filtration of an insoluble re=sidue, provided by addition of n-hexane a white crystalline solid having a M.P.: 185-187°C.

IR was consistent with the assigned structure.

EXAMPLE 12

Preparation of N-[(4,6-dimethyl-2-pyrimidyl)aminocarbonyl]--2,3-dihydro-2,2,5-trimethyl-7-benzofuransulfonamide

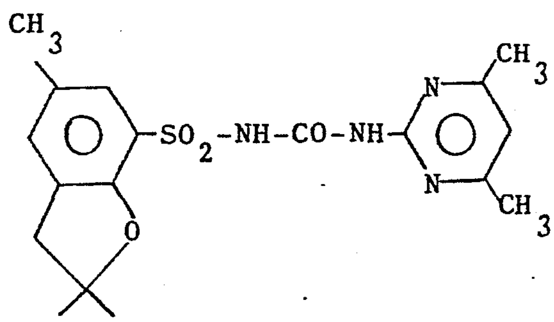

A solution consisting of 1.5 g of 2,3-dihydro-2,2,5-trime=thyl-7-benzofuransulfonylisocyanate in 3 ml of anhydrous ace=tonitrile was added slowly at room temperature to a well stirred suspension of 0.6 g of 2-amino-4,6-dimethyl-pyrimi=dine in 10 ml of anhydrous acetonitrile containing 0.02 g of triethylamine.

The reaction mixture was maintained at room temperature under strong stirring for three hours. After cooling down to 2°C a precipitate was separated, that was collected by filtra=tion and washed with a mixture of toluene/n-hexane (1/1) and dried in the air.

The thus obtained product consisted of 1.4 g of a white crystalline solid having a M.P.: 171-173°C.

IR was consistent with the assigned structure.

EXAMPLE 13

Preparation of N-[(4-methoxy-6-methyl-1,3,5-triazin-2-il) aminocarbonyl]-2,3-dihydro-2-methyl-5-chloro-7-benzofuran=sulfonamide

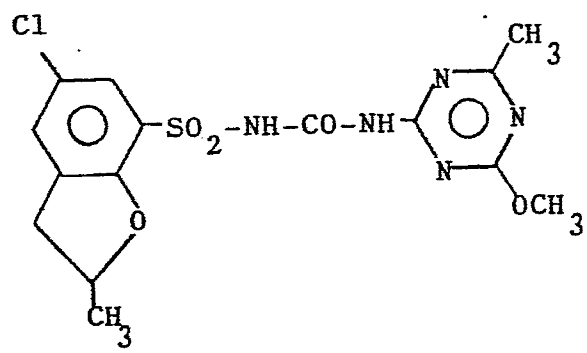

A solution consisting of 1.5 g of 2,3-dihydro-2-methyl-5-chloro-7-benzofuransulfonylisocyanate in 5 ml of anhydrous acetonitrile was added at room temperature to a well stirred suspension of 0.7 g of 2-amino-4-methoxy-6-methyl-1,3,5-triazine in 10 ml of anhydrous acetonitrile containing 0.02 g of triethylamine.

The mixture was kept under good stirring at room temperature for thirty hours, then the precipitate was separated by fil=tration and washed with acetonitrile. The thus obtained product consisted of 1.4 g of a white crystalline solid ha=ving a M.P.: 200-202°C.

IR was consistent with the assigned structure.

EXAMPLE 14

Determination of the herbicide activity during post-emergen= ce.

Small pots were prepared containing sandy earth (sizes: height = 10 cm, top diameter = 10 cm).

In each pot one of the following weeds was sown:

*Echinochloa crusgalli*

*Avena fatua*

*Setaria glauca*

*Digitaria sanguinalis*

*Stellaria media*

*Ipomea spp.*

*Vigna sinensis*

*Galium aparine*

*Rumex acetosella*

*Veronica persica*

Water in an amount as required to ensure a good germination of the seeds was added to each pot. The pots were then divi= ded in two sets.

The first set was not treated with any weed-killer and was used as a comparison test (control).

The second set was treated 15 days after the sowing (i.e. when the small plants, depending on the species, had already reached a height of 5 to 10 cm) with a water-acetone disper=

sion (20% by volume of acetone) of the compounds of the invention in order to evaluate the herbicide activity during Post-emergence.

During the whole period of time in which the tests were performed, the small pots were kept in a conditioned environment at temperatures ranging between 15 and 24°C, relative humidity = 70%, photoperiod = 12 hours and luminous intensity = 2500 lux.

Every two days the pots were uniformly watered, in order to ensure a humidity degree sufficient for a good development of the plants.

28 days after the treatment with the compound according to the invention the vegetative stage of the plants was surveyed. The herbicide activity of the compounds of the invention was evaluated by comparing the growth and the vegetative stage of the treated plants with respect to the non-treated plants (control) and it was expressed according to a scale of values from 0 (herbicide activity = 0, growth equal to the one of control) to 4 (complete stop of the growth or death of the plant), the intermediate values represent intermediate situations of vegetative development.

Compounds of the invention hexibited a complete activity (4) against the above mentioned weeds at a dose of 1 Kg/ha, maintaining a complete or very high activity at lower doses as well.

In the above formulae the radicals are defined as follows (see e.g. also page 3):

alkyl: methyl, ethyl, propyl, i-propyl, butyl, i-butyl, sec.-butyl, tert.-butyl

alkenyl: vinyl, propenyl-1 or -2, the butenyl and pentenyl residues

alkynyl: acetynyl, propynyl-1 or -2, the butynyl and pentynyl residues

phenyl groups may be substituted by 1, 2 or 3 of the stated substituents and as far halogen is a substituent in O-alkyl groups etc., also 1,2 or 3 halogen atoms which may be equal or different may be present

aryl is preferably a phenyl group

The above given definition for alkyl is also valid for more complex groups such as alkoxy or COO alkyl. In O-alkenyl and alkynyl alkenyl and alkynyl are defined as above.

CLAIMS

1. Compounds of formula

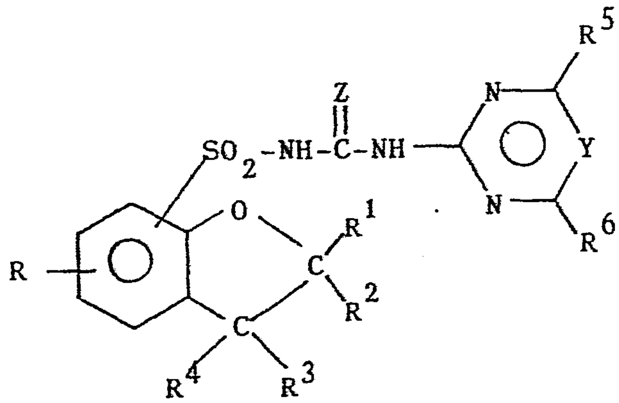

(I)

wherein

R represents a hydrogen atom, a halogen atom, a methyl, a nitro, a COO-alkyl, a $SO_2$-alkyl, an aminocarbonyl, a mono- or di-alkylaminocarbonyl group, an O-alkyl group optionally substituted by from 1 to 3 halogen atoms, an O-alkenyl group optionally substituted by from 1 to 3 halogen atoms, an O-alkynyl, an O-aryl, or a $NR^7$-CO-$R^8$ group, wherein $R^7$ and $R^8$ are independently a hydrogen atom, an alkyl or phenyl group or together they form a trimethylenic or tetramethylenic bridge;

$R^1$, $R^2$, $R^3$ and $R^4$, equal to or different from each other, represent a hydrogen atom or a methyl group; or

$R^1$ and $R^2$ together with the carbon atom, they are linked to, form a carbonyl group (C=O) and/or $R^3$ and $R^4$ together with the carbon atom, they are linked to, form a carbonyl group (C=O);

$R^5$ and $R^6$, equal to or different from each other, repre= sent a methyl or a methoxy group;

Z represents an oxygen or a bivalent sulphur atom;

Y represents a nitrogen atom or a CH group.

2. Compounds according to claim 1, having formula

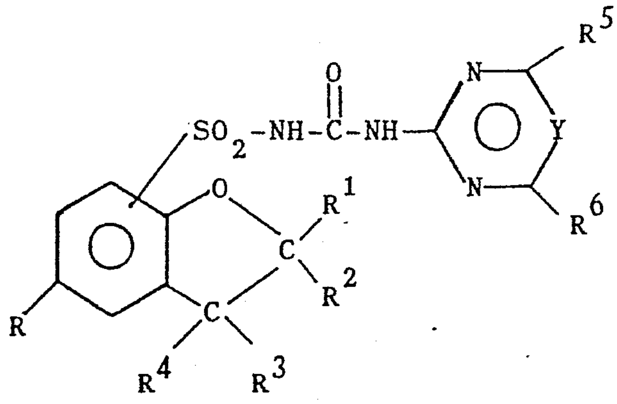

wherein

R, $R^1$, $R^2$, $R^3$, $R^4$, $R^5$, $R^6$ and Y have the same meanings recorded in claim 1.

3. Compounds according to claim 2 wherein R is selected among a chlorine atom, a methyl or a nitro group.

4. Compound N-[(4-methoxy-6-methyl-1,3,5-triazin-2-il)-amino= carbonyl]-2,3-dihydro-2,2-dimethyl-5-chloro-7-benzofuran= sulfonamide.

5. Compound N-[(4,6-dimethyl-2-pyrimidyl)-aminocarbonyl]-2,3-dihydro-2,2-dimethyl-5-chloro-7-benzofuransulfonamide.

6. Compound N-[(4-methoxy-6-methyl-1,3,5-triazin-2-il)-amino= carbonyl]-2,3-dihydro-2,2,5-trimethyl-7-benzofuransulfona= mide.

7. Compound N-[(4,6-dimethyl-2-pyrimidyl)-aminocarbonyl]-2,3-dihydro-2,2,5-trimethyl-7-benzofuransulfonamide.

8. Compound N-[(4-methoxy-6-methyl-1,3,5-triazin-2-il)-amino=carbonyl]-2,3-dihydro-2-methyl-5-chloro-7-benzofuransulfo=namide.

9. Compounds of formula:

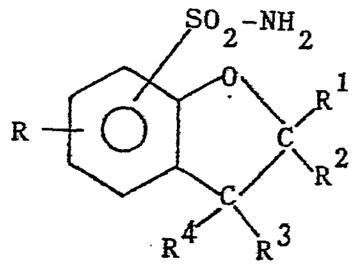

(V)

wherein

R, $R^1$, $R^2$, $R^3$ and $R^4$ have the same meanings recorded in claim 1.

10. A process for the preparation of the compounds of claim 1 consisting in reacting, in an inert solvent and in the presence of a catalytic amount of a tertiary amine, a com=pound of formula

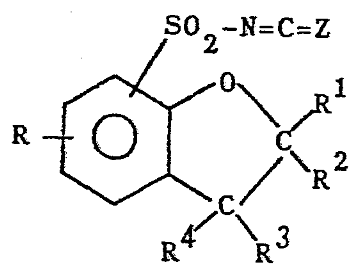

(VI)

(wherein R, $R^1$, $R^2$, $R^3$, $R^4$ and Z have the same meanings recorded in claim 1) with an aromatic amine of formula:

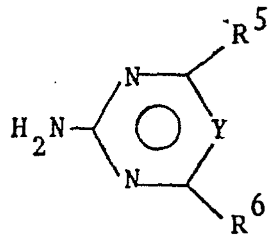

(VII)

(wherein $R^5$, $R^6$ and Y have the same meanings recorded in claim 1).

11. A method for fighting weeds in cultivations of agrarian interest consisting in distributing on the soil or on the weed an effective amount of one or more compounds according to claim 1, as such or in the form of a suitable composition.

12. The method of claim 11 used in the herbicide treatment of post-emergence.

13. A herbicide composition containing as active substance one or more compounds of claim 1, together with inert carriers and optionally other additives.

European Patent Office

# EUROPEAN SEARCH REPORT

0123303

Application number

EP 84 10 4510

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 045 196 (E.I. DU PONT DE NEMOURS AND COMPANY) * Claims 1,18,24 * | 1,9,11 | C 07 D 405/12<br>C 07 D 307/79<br>A 01 N 47/36 |
| A | EP-A-0 030 142 (E.I. DU PONT DE NEMOURS AND COMPANY) * Claims * | 1,11 | |
| A | EP-A-0 044 807 (CIBA-GEIGY AG) * Claims * | 1,11 | |
| E | EP-A-0 099 339 (CIBA-GEIGY AG) * Claims; examples 1,3; tables 1,5, compounds no. Q1-Q5 * | 1,2,9, 11 | |
| E | EP-A-0 079 683 (I.E. DU PONT DE NEMOURS AND COMPANY) * Examples 2,5; pages 101-107; claim 16 * | 1,2,8, 11 | TECHNICAL FIELDS SEARCHED (Int. Cl. 3)<br>C 07 D 405/00<br>C 07 D 307/00 |

The present search report has been drawn up for all claims

| Place of search | Date of completion of the search | Examiner |
|---|---|---|
| THE HAGUE | 19-07-1984 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82